# EUROPÄISCHE PATENTSCHRIFT

(11) **EP 1 518 117 B1**
(45) Veröffentlichungstag und Bekanntmachung des Hinweises auf die Patenterteilung: **28.02.2007**
(21) Anmeldenummer: 03759971.9
(22) Anmeldetag: 18.06.2003
(51) Int. Cl.: G01N 33/50

(54) **VERFAHREN ZUR DIAGNOSE UND BEHANDLUNG VON NIS-EXPRIMIERENDEN KARZINOMEN UND METASTASEN**
METHOD FOR DIAGNOSING AND TREATING NIS-EXPRESSING CARCINOMAS AND METASTASES
METHODE DE DIAGNOSTIC ET DE TRAITEMENT DE METASTASES ET DE CARCINOMES EXPRIMANT NIS

(30) Priorität: 18.06.2002 DE 10227136
(43) Veröffentlichungstag der Anmeldung: 30.03.2005
(73) Patentinhaber: Loos, Ulrich, 89081 Ulm (DE)
(72) Erfinder: Loos, Ulrich, 89081 Ulm (DE)
(74) Vertreter: Patentanwälte Ruff, Wilhelm, Beier, Dauster & Partner
(86) Internationale Anmeldenummer: PCT/EP2003/006435
(87) Internationale Veröffentlichungsnummer: WO 2003/107001

(56) Entgegenhaltungen:
- WO-A-02/055688
- DE-A- 19 805 788
- KOGAI T ET AL: "Retinoic acid induces sodium/iodide symporter gene expression and radioiodide uptake in the MCF-7 breast cancer cell line." PROCEEDINGS OF THE NATIONAL ACADEMY OF SCIENCES OF THE UNITED STATES OF AMERICA. UNITED STATES 18 JUL 2000, Bd. 97, Nr. 15, 18. Juli 2000 (2000-07-18), Seiten 8519-8524, XP002258675 ISSN: 0027-8424 in der Anmeldung erwähnt
- SPITZWEG C ET AL: "Treatment of prostate cancer by radioiodine therapy after tissue-specific expression of the sodium iodide symporter." CANCER RESEARCH. UNITED STATES 15 NOV 2000, Bd. 60, Nr. 22, 15. November 2000 (2000-11-15), Seiten 6526-6530, XP002258676 ISSN: 0008-5472
- SPITZWEG C ET AL: "In vivo sodium iodide symporter gene therapy of prostate cancer." GENE THERAPY. ENGLAND OCT 2001, Bd. 8, Nr. 20, Oktober 2001 (2001-10), Seiten 1524-1531, XP002258677 ISSN: 0969-7128
- SCHMUTZLER C: "Regulation of the sodium/iodide symporter by retinoids: A review." EXPERIMENTAL AND CLINICAL ENDOCRINOLOGY & DIABETES, Bd. 109, Nr. 1, 2001, Seiten 41-44, XP009018335 ISSN: 0947-7349
- MEHTA RAJENDRA G ET AL: "A ligand of peroxisome proliferator-activated receptor gamma, retinoids, and prevention of preneoplastic mammary lesions." JOURNAL OF THE NATIONAL CANCER INSTITUTE (BETHESDA)., Bd. 92, Nr. 5, 1. März 2000 (2000-03-01), Seiten 418-423, XP009018462 ISSN: 0027-8874
- ELSTNER E ET AL: "Ligands for peroxisome proliferator-activated receptorgamma and retinoic acid receptor inhibit growth and induce apoptosis of human breast cancer cells in vitro and in BNX mice" PROCEEDINGS OF THE NATIONAL ACADEMY OF SCIENCES OF USA, NATIONAL ACADEMY OF SCIENCE. WASHINGTON, US, Bd. 95, Juli 1998 (1998-07), Seiten 8806-8811, XP000909123 ISSN: 0027-8424
- SATO M ET AL: "Synergistic potentiation of thiazolidinedione-induced ST 13 preadipocyte differentiation by RAR synergists." BIOCHEMICAL AND BIOPHYSICAL RESEARCH COMMUNICATIONS. UNITED STATES 26 JAN 2001, Bd. 280, Nr. 3, 26. Januar 2001 (2001-01-26), Seiten 646-651, XP002258678 ISSN: 0006-291X
- SCHULMAN IRA G ET AL: "Transactivation by retinoid X receptor-peroxisome proliferator-activated receptor gamma (PPARgamma) heterodimers: Intermolecular synergy requires only the PPARgamma hormone-dependent activation function" MOLECULAR AND CELLULAR BIOLOGY, Bd. 18, Nr. 6, Juni 1998 (1998-06), Seiten 3483-3494, XP002258679 ISSN: 0270-7306
- WESTIN STEFAN ET AL: "Interactions controlling the assembly of nuclear-receptor heterodimers and co-activators" NATURE (LONDON), Bd. 395, Nr. 6698, 10. September 1998 (1998-09-10), Seiten 199-202, XP002258680 ISSN: 0028-0836
- KOGAI T ET AL: "Differential regulation of the human sodium/iodide symporter gene promoter in papillary thyroid carcinoma cell lines and normal thyroid cells." ENDOCRINOLOGY. UNITED STATES AUG 2001, Bd. 142, Nr. 8, August 2001 (2001-08), Seiten 3369-3379, XP002258681 ISSN: 0013-7227
- FILETTI S ET AL: "Sodium/iodide symporter: a key transport system in thyroid cancer cell metabolism." EUROPEAN JOURNAL OF ENDOCRINOLOGY / EUROPEAN FEDERATION OF ENDOCRINE SOCIETIES. ENGLAND NOV 1999, Bd. 141, Nr. 5, November 1999 (1999-11), Seiten 443-457, XP002258682 ISSN: 0804-4643
- KITAZONO M ET AL: "Low concentrations of the histone deacetylase inhibitor, depsipeptide (FR901228), increase expression of the Na+/I- symporter and iodine accumulation in poorly differentiated thyroid carcinoma cells" JOURNAL OF CLINICAL ENDOCRINOLOGY AND METABOLISM, NEW YORK, NY, US, Bd. 86, Nr. 7, Juli 2001 (2001-07), Seiten 3430-3435, XP002225997 ISSN: 0021-972X
- TANOSAKI SAKAE ET AL: "Effect of ligands of nuclear hormone receptors on sodium/iodide symporter expression and activity in breast cancer cells." BREAST CANCER RESEARCH AND TREATMENT, Bd. 79, Nr. 3, 20. Juni 2003 (2003-06-20), Seiten 335-345, XP009018337 ISSN: 0167-6806

## Beschreibung

Die vorliegende Erfindung betrifft Verwendungen zur Herstellung eines Diagnostikmittels und Medikaments zum Nachweis bzw. zur Behandlung von Natrium-Jodid-Symporter (NIS) exprimierenden primären Karzinomen und Metastasen, vorzugsweise von glandulären Karzinomen, insbesondere von Karzinomen der Schilddrüse, der Speicheldrüse, des Uterus und von Mammakarzinomen, entsprechende Verfahren zur *in vitro-*Diagnose sowie ein Kombinationspräparat in Form eines Kits enthaltend Substanzen, welche die Expression bzw. die Funktion von NIS induzieren und/oder steigern und als Folge die Jodid-Aufnahme in die Zellen verstärken. Dies wird für eine effektive organspezifische Radiojodid-Aufnahme in der Diagnostik und Therapie von genannten Karzinomen und Metastasen verwendet.

Knoten gutartig ist. Durch die aufwendige Diagnostik kommt es häufig zu Zeitverlust und unnötigen operativen Eingriffen.

Die primäre Therapie der genannten Tumore besteht in der Regel in der operativen Resektion des Primärkarzinoms. Je nach Größe des Tumors (z.B. größer als 2 cm im Durchmesser) oder Hinweisen auf Lymphknotenbefall erfolgt insbesondere beim Mammakarzinom zusätzlich die Ausräumung der Lymphknoten der gleichseitigen Achselhöhle, gegebenenfalls auch eine Bestrahlung des überregionalen Gebietes. Postoperativ erfolgen Untersuchungen zum Nachweis oder Ausschluss von Fernmetastasen, d.h. beispielsweise eine Oberbauchsonographie, eine Röntgen-Thorax-Aufnahme und ein Skelett-Szintigramm. Dabei werden nur Metastasen ab einer gewissen Größe erkannt. Kleinere Metastasen, also beispielsweise kleiner als 0,5 cm im Durchmesser, sogenannte Mikrometastasen, werden oftmals nicht erkannt. Beim Knochenszintigramm können degenerative oder entzündliche Veränderungen zu falsch positiven Befunden führen. Im Falle von Metastasen erfolgt eine Chemotherapie, die erhebliche Nebenwirkungen haben kann. Diese Behandlung ist nur palliativ und führt lediglich zu einer geringen Lebenszeitverlängerung.

Eine Aufgabe der vorliegenden Erfindung besteht darin, ein Verfahren zur schnellen, spezifischen und zuverlässigen Diagnose sowie zur effektiven Behandlung von Karzinomen und Metastasen, insbesondere von primären glandulären Karzinomen und Metastasen glandulärer Karzinome, insbesondere von Karzinomen der Schilddrüse, der Speicheldrüse, des Uterus und von Mammakarzinomen, eine pharmakologische Zusammensetzung zur Therapie sowie ein in vitro-Diagnosesystem an reseziertem Gewebe zur Diagnose und Beurteilbarkeit der Therapiemöglichkeit genannter Karzinome bereit zu stellen.

Zur Lösung dieser Aufgabe schlägt die Erfindung in erster Linie ein Verfahren mit den im Anspruch 1 genannten Merkmalen vor. Weiterbildun-Eine Aufgabe der vorliegenden Erfindung besteht darin, Verwendungen zur schnellen, spezifischen und zuverlässigen Diagnose sowie zur effektiven Behandlung von Karzinomen und Metastasen, insbesondere von primären glandulären Karzinomen und Metastasen glandulärer Karzinome, insbesondere von Karzinomen der Schilddrüse, der Speicheldrüse, des Uterus und von Mammakarzinomen, ein *in vitro*-Diagnosesystem an reseziertem Gewebe zur Diagnose und Beurteilbarkeit der Therapiemöglichkeit genannter Karzinome sowie ein Kombinationspräparat in Form eines Kits bereit zu stellen.

Zur Lösung dieser Aufgabe schlägt die Erfindung in erster Linie Verwendungen mit den in den Ansprüchen 1 und 2 genannten Merkmalen vor. Weiterbildungen hierzu sind Gegenstand der Ansprüche 2 bis 11. Die Ansprüche 12 und 13 betreffen jeweils ein Verfahren zur *in vitro-*Diagnose. Die Ansprüche 14 bis 24 beziehen sich auf ein Kombinationspräparat in Form eines Kits. Der Wortlaut sämtlicher Ansprüche wird durch Bezugnahme zum Inhalt der Beschreibung gemacht.

Das oben genannte Ziel wird gemäß der Erfindung durch ein Verfahren erreicht, bei dem durch eine Induktion bzw. Steigerung der NIS-Genexpression und -Funktion in den genannten Tumorarten eine verstärkte Jodid-Aufnahme organspezifisch stimuliert wird, die zu einer spezifischen Diagnose und/oder einer gezielten Therapie genutzt werden kann.

Der Natrium-Jodid-Symporter (NIS) ist für die aktive Jodid-Aufnahme in der Schilddrüse verantwortlich. Das Jod wird in den Follikelzellen der Schilddrüse zum Zwecke der Biosynthese der Schilddrüsenhormone gebraucht (N. Carrasco, Biochim Biophys Acta. 1993 Jun 8; 1154 (1): 65-82). Die Expression von NIS ist über Jahrzehnte für die Diagnose und Behandlung der differenzierten Schilddrüsenkarzinome basierend De La Vieja et al., Physiol Rev. 2000 Jul. 80 (3):1083-105; C. Riedel et al., Trends Biochem Sci. 2001 Aug. 26 (8):490-6; A.E. Heufelder et al., Thyroid. 2001 Sep. 11 (9): 839-47).

Faktoren, die die NIS-Expression und die Jodid-Aufnahme in Brustkrebszellen modulieren, sind bisher nicht im Detail untersucht worden. Kürzlich ist über die Induktion des NIS-Gens durch *trans*-Retinsäure (tRA) in der Brustkrebs-Zelllinie MCF-7 berichtet worden. Es wurde gemutmaßt, dass die tRA-induzierte NIS-Expression durch zwei Familien von nukleären Rezeptoren, nämlich Retinsäure-Rezeptoren (RARs) und Retinoid-X-Rezeptoren (RXRs) stimuliert wird (T. Kogai et al., Proc Natl Acad Sci USA. 2000 Jul. 97 (15): 8519-24). MCF-7-Zellen exprimieren neben funktionalen RAR/RXR auch nukleäre PPAR-γ (Peroxisome Proliferator Activated Receptor-γ) (M.W. Titcomb et al., Mol Endocrinol. 1994 Jul. 8 (7): 870-7; M.W. Kilgore et al., Mol Cell Endocrinol. 1997 May 129 (2): 229-35). PPAR-γ reguliert in Gegenwart von entsprechenden Liganden durch Heterodimerisierung mit RAR/RXR (RXRα, RXRβ oder RXRγ) die Transkription von Zielgenen (M. Sato et al., Biochem Biophys Res Commun. 2001 Jan. 280 (3): 646-51). PPAR-γ und RAR/RXR werden in verschiedenen bösartigen epithelialen Tumoren wie Gehirn-, Brust-, Prostata- und Lungenkarzinomen koexprimiert (J.O. Nwankwo and M.E. Robbins, Prostaglandins Leukot Essent Fatty Acids. 2001 Apr.-May 64 (4-5): 241-5; K. Inoue et al., Anticancer Res. 2001 Jul.-Aug. 21 (4A): 2471-6).

Die vorliegende Erfindung zeigt, dass eine Jodid-Aufnahme in Zellen bestimmter Karzinome durch eine gleichzeitige oder zeitversetzte Einwirkung von PPAR-γ-Liganden und RAR/RXR-Liganden erheblich gesteigert werden kann, wobei die PPAR-γ-Liganden die durch die RAR/RXR-Liganden induzierte Jodid-Aufnahme um ein vielfaches potenzieren. Dieser Synergismus ist auf eine Heterodimerisierung der Rezeptoren PPAR-γ und RAR/RXR und eine dadurch induzierte bzw. sti-die Transkription von Zielgenen (M. Sato et al., Biochem Biophys Res Commun. 2001 Jan. 280 (3): 646-51 i I.G. Schulmann et al. (1998), Mol. Cell. Biol., Bd. 18, No. 6, S. 3483 - 3494). PPAR-γ und RAR/RXR werden in verschiedenen bösartigen epithelialen Tumoren wie Gehirn-, Brust-, Prostata- und Lungenkarzinomen koexprimiert (J.O. Nwankwo and M.E. Robbins, Prostaglandins Leukot Essent Fatty Acids. 2001 Apr.-May 64 (4-5): 241-5; K. lnoue et al., Anticancer Res. 2001 Jul.-Aug. 21 (4A): 2471-6).

Aus der DE 198 05 788 A1 ist die Klonierung, Sequenzierung und Charakterisierung des NIS-GEN-Promotors sowie dessen mögliche Verwendung in der Radiojodtherapie bekannt. Ebenso sind bereits Gentherapien auf der Basis einer Transfektion mit exogener NIS-DNA zur Behandlung von Prostatakrebs im Rahmen einer Radiojodtherapie bekannt (C. Spitzweg et al., Cancer Research 2000, Nov. 60: 6526-6530; C. Spitzweg et al, Gene Therapy 2001, 8: 1524-1531). Dem Review-Artikel von C. Schmutzler kann die Verwendung von Retinsäure und ihren Derivaten für die Radiojodtherapie von Schilddrüsen- und Brustkrebs entnommen werden (C. Schmutzler, Experimental and Clinical Endocrinology & Diabetes 109 (2001): 41-44). Weiterhin ist aus dem Stand der Technik bekannt, dass die PPAR-γ-Liganden und die RAR- bzw. RXR-Liganden eine Inhibierung der Zellproliferation und eine Stimulation der Apoptose bewirken (Mehta RG et al. (2000), J. Nat. Canc. Inst., Bd. 92, Nr. 5, S. 418, 423 und Elstner E et al. (1998), Proc. Natl. Acad. Sci., Bd. 95, S. 8806-8811). Ferner liegen mechanistische Untersuchungen zu PPAR-γ-RXR- bzw. RAR/RXR-Heterodimeren und entsprechenden Hormon-Rezeptor-Liganden vor (Westin S. et al. (1998), Nature, Bd. 395, S. 199-202). Aus der WO 02/055 688 A2 geht die Verwendung von Histon-Deacetylase-Inhibitoren zur Detektion und/oder Behandlung von Schilddrüsenkarzinomen hervor.

Die vorliegende Erfindung zeigt, dass eine Jodid-Aufnahme in Zellen bestimmter Karzinome durch eine gleichzeitige oder zeitversetzte Einwirkung von PPAR-γ-Liganden und RAR/RXR-Liganden erheblich gesteigert werden kann, wobei die PPAR-γ-Liganden die durch die RAR/RXR-Liganden induzierte Jodid-Aufnahme um ein vielfaches potenzieren. Dieser Synergismus ist auf eine Heterodimerisierung der Rezeptoren PPAR-γ und RAR/RXR und eine dadurch induzierte bzw. stimulierte und/oder verstärkte Expression des NIS-Gens zurückzuführen. Eine hierdurch bewirkte Steigerung der Jodid-Aufnahme in die Zelle wird erfindungsgemäß dazu genutzt, NIS-exprimierende primäre Karzinome und Metastasen, insbesondere Primärtumore und/oder Metastasen glandulärer Karzinome, insbesondere von Karzinomen der Schilddrüse, der Speicheldrüse, des Uterus und von Mammakarzinomen sowie anderen glandulären Karzinomen, zu diagnostizieren und zu therapieren.

Zum Nachweis der Expressionsinduktion des NIS-Gens in den ausgewählten Karzinomen durch die heterodimerisierenden Rezeptoren PPAR-γ und RAR/RXR wurde in der vorliegenden Erfindung insbesondere die Wirkung von beispielsweise Ciglitazon (CIG), einem synthetischen Liganden des PPAR-γ, auf die Jodid-Aufnahme durch MCF-7-Zellen mit und ohne Zugabe von *trans-*RA (tRA) untersucht. Die Brustkrebs-Zelllinie MCF-7 exprimiert die Rezeptoren PPAR-γ und RAR/RXR (M.W. Kilgore et al., Mol Cell Endocrinol. 1997 May 129 (2): 229-35). Erfindungsgemäß konnte gezeigt werden, dass die durch die entsprechenden Liganden aktivierten Rezeptoren die Expression des NIS-Gens synergistisch induzieren bzw. stimulieren und/oder verstärken und damit die Funktion von NIS verstärken.

Ciglitazon (CIG) gehört zur Substanzklasse der synthetischen Thiazolidindione (TZDs), welche die potentesten Induktoren von PPAR-γ darstellen (G.J. Murphy and J.C. Holder, Trends Pharmacol Sci. 2000 Dec. 21 (12): 469-74). Diese Substanz wurde als selektiver Aktivator von PPAR-γ beschrieben (Y. Sugiyama et al., Nippon Rinsho. 2000 Feb. 58 (2): 370-5).

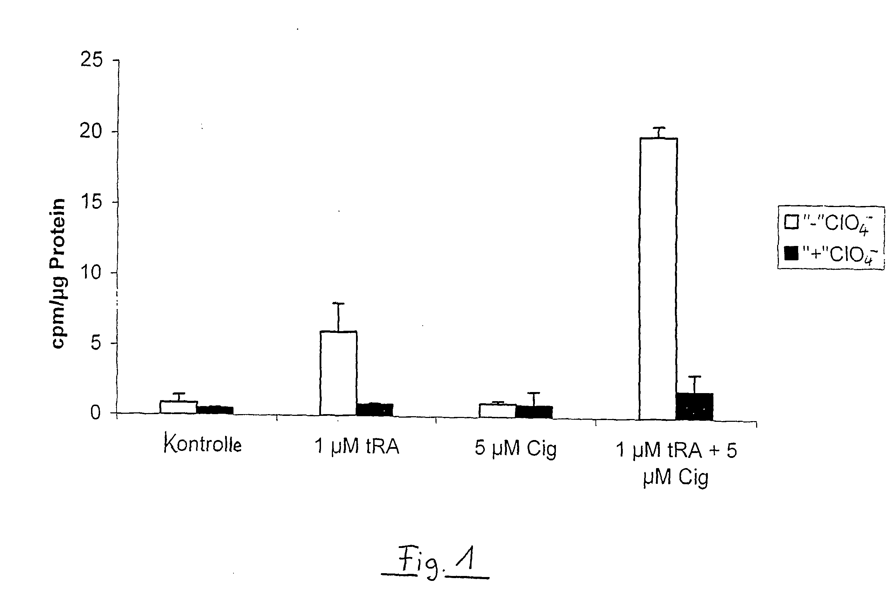

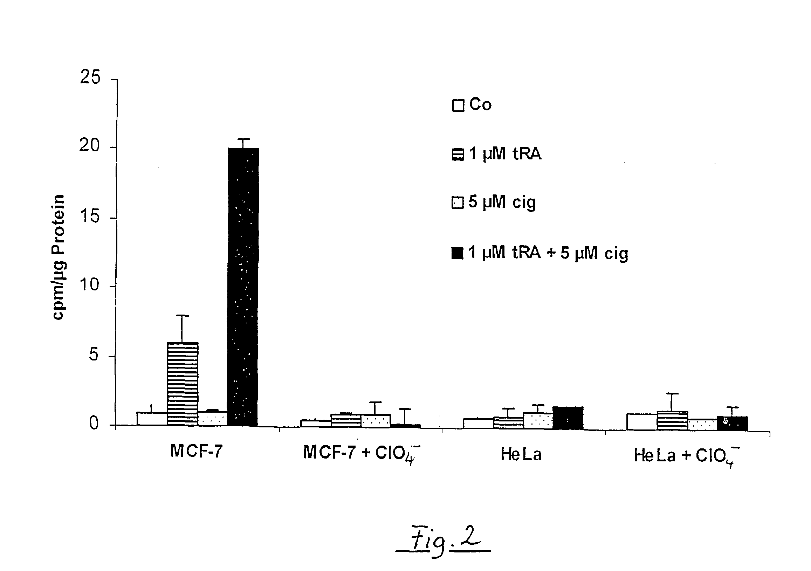

Die vorliegende Erfindung zeigt, dass CIG in Kombination mit tRA die Jodid-Aufnahme in der Brustkrebs-Zelllinie MCF-7 im Vergleich mit der durch tRA allein induzierten bzw. gesteigerten Jodid-Aufnahme deutlich verstärkt, während CIG alleine keine Wirkung zeigt (**Fig. 1**). Die durch CIG und tRA induzierte NIS-Expression bzw. die dadurch bewirkte Jodid-Aufnahme ist spezifisch für MCF-7-Zellen. Beispielsweise in der Zervixkarzinom-Zelllinie HeLa konnte nach einer Behandlung über 48 Stunden mit CIG und tRA keine Jodid-Aufnahme beobachtet werden (**Fig. 2**).

Die Behandlung von MCF-7-Zellen mit tRA allein steigert die Jodid-Aufnahme um ca. das 6-fache. Die Kombination von tRA mit CIG steigert jedoch die Jodid-Aufnahme um ca. das 20-fache im Vergleich zu unbehandelten Zellen (**Fig. 1**). Eine gleichzeitige Behandlung der Brustkrebszellen mit CIG und tRA bewirkt somit einen mehr als 3-fachen Anstieg in der Jodid-Aufnahme im Vergleich zu nur mit tRA behandelten Zellen. CIG alleine induziert die Jodid-Aufnahme in MCF-7-Zellen nicht. Weiterhin wurde erfindungsgemäß gezeigt, dass durch eine Behandlung der Zellen mit tRA die Konzentration von NIS-mRNA deutlich ansteigt, was eine Wirkung der Substanzen auf der Transkriptionsebene belegt. Diese Ergebnisse zeigen, dass die NIS-Genexpression synergistisch durch die Behandlung von CIG in Kombination mit Retinsäure in der Brustkrebs-Zelllinie MCF-7 gesteigert wird.

Aus früheren Untersuchungen ist bekannt, dass *trans*-Retinsäure (tRA) die Jodid-Aufnahme in MCF-7-Zellen induziert, indem es die NIS-Expression stimuliert, wobei die Wirkung von tRA durch die Rezeptoren RAR/RXR vermittelt wird (T. Kogai et al., Proc Natl Acad Sci USA. 2000 Jul. 97 (15): 8519-24). Die Ergebnisse, die zur vorliegenden Erfindung geführt haben, zeigen das Vorliegen von funktionalen responsiven Elementen für die Rezeptoren PPAR-γ und RAR/RXR in Krebszellen, wobei die responsiven Elemente bzw. die Rezeptoren synergistisch miteinander wechselwirken. Die Beobachtung, dass die durch eine gleichzeitige Einwirkung von CIG und tRA induzierte Zunahme der NIS-Expression spezifisch für MCF-7-Zellen ist und nicht in beispielsweise HeLa-Zellen stattfindet (**Fig. 2**), zeigt die Selektivität des beschriebenen Mechanismus für bestimmte Krebszellen. Dieses Ergebnis steht im Einklang mit der bereits beschriebenen Beobachtung, dass tRA die Jodid-Aufnahme der bereits beschriebenen Beobachtung, dass tRA die Jodid-Aufnahme in anderen Krebs-Zelllinien, wie der Prostatakrebs-Zelllinie LNCaP, der Choriokarzinom-Zellinie JEG-3 oder zwei nicht kleinzelligen Lungenkrebs-Zellinien A549 und H460 nicht induziert (T. Kogai et al., Proc Natl Acad Sci USA. 2000 Jul. 97 (15): 8519-24).

Auf der Basis der beschriebenen Ergebnisse wird ein Verfahren zur Diagnose und/oder Behandlung von Natrium-Jodid-Symporter (NIS) exprimierenden Karzinomen und/oder Metastasen vorgeschlagen. Bei diesem Verfahren wird eine Induktion bzw. Steigerung der NIS-Genexpression in den genannten Tumorarten und eine verstärkte Jodid-Aufnahme organspezifisch stimuliert, die zu einer spezifischen Diagnose und/oder einer gezielten Therapie erfindungsgemäß genutzt wird.

Besonders bevorzugt kann das Verfahren für die Diagnose und/oder Behandlung von glandulären Karzinomen, insbesondere Karzinomen der Speicheldrüse, der Schilddrüse, des Uterus und von Brustkrebs bzw. Mammakarzinomen, und/oder den entsprechenden Metastasen eingesetzt werden.

Die induzierte bzw. gesteigerte NIS-Genexpression wird erfindungsgemäß dazu genutzt, durch die verstärkte Expression bzw. Funktion des Natrium-Jodid-Symporters bevorzugt radioaktive Substanzen in die Tumorzellen einzuschleusen. Die Radioaktivität ist als ein sehr sensitiver Indikator bei der Diagnostik mittels szintigraphischer Verfahren und/oder als aktives therapeutisches Mittel bei der Therapie mittels Radiolyse nutzbar. Unter Radiolyse ist die Zerstörung der Zellen durch radioaktive Strahlung zu verstehen. Ein besonderer Vorteil der Radiolyse ist, dass auch bei einer nur geringen Aufnahme von Radiojod die radiolytische Wirkung aufgrund der Reichweite der Strahlung auf auf benachbarte Zellen als sogenannter By-Stander-Effekt übertragen wird.

Erfindungsgemäß werden Substanzen bevorzugt eingesetzt, die eine hohe Affinität zum Symporter NIS aufweisen, insbesondere Jod und/oder Technetium. Aber auch andere affine Substanzen, insbesondere aus der Gruppe der Halogene, können Verwendung finden. Die Anwendung von Radiojodid und/oder Technetium, insbesondere radioaktivem Technetium, nach vorzugsweise systemischem Einsatz der NIS-Gen-induzierenden Wirkstoffe, also insbesondere der PPAR-γ- und RAR/RXR-Liganden, erweist sich erfindungsgemäß als ein besonders wertvolles Mittel zur Diagnose und Behandlung der oben angegebenen Tumoren. Besonders vorteilhaft können hierfür als radioaktives Jod ¹²³I, ¹²⁵I und/oder ¹³¹I, insbesondere in Form eines Alkali- oder Erdalkalijodids, beispielsweise Natriumjodid (Nal), verwendet werden. Hierbei wird ¹²⁵I mit besonderem Vorteil für eine Diagnostik und ¹³¹I mit besonderem Vorteil für eine Therapie eingesetzt.

Im Folgenden wird unter dem Begriff "Jodid-Aufnahme" auch die Aufnahme anderer Substanzen verstanden, die eine Affinität zum Symporter NIS aufweisen und von ihm aktiv transportiert werden können. Die Zugabe der Substanz, deren Transport durch den Natrium-Jodid-Symporter nachgewiesen werden soll oder die eine Radiolyse verursachen soll, erfolgt vorteilhafterweise nach einer angemessenen Zeit nach Verabreichung der Wirkstoffe, die der Stimulierung und/oder Verstärkung der NIS-Genexpression dienen. Eine angemessene Zeit kann hierbei beispielsweise etwa 1 bis 5 Tage betragen, vorzugsweise etwa 1 bis 3 Tage. Besonders bevorzugt sind etwa 2 Tage.

In einer bevorzugten Ausführungsform des Verfahrens erfolgt die Induktion der NIS-Genexpression mittels einer Behandlung mit mindestens einem PPAR-Liganden, insbesondere einem PPAR-γ-Liganden, und mindestens einem RAR/RXR-Liganden als Wirkstoffe, wobei die durch den RAR/RXR-Liganden gesteigerte Jodid-Aufnahme durch den PPAR-γ-Liganden in synergistischer Weise potenziert wird. Besonders bevorziert wird. Besonders bevorzugt werden dabei die Liganden für die beiden Rezeptorarten zeitlich versetzt verabreicht, wobei vorzugsweise der mindestens eine RAR/RXR-Ligand zuerst und der mindestens eine PPAR-Ligand nach einer angemessenen Zeit appliziert wird. In dieser Zeit soll eine anfängliche Induktion der NIS-Gen-Expression erfolgen. Weiterhin kann durch die zeitversetzte Applikation eine Blockierung des PPAR-γ, die durch einen Suppressor bewirkt sein kann, durch einen mit einem entsprechenden Liganden aktivierten RAR/RXR augehoben werden, so dass der PPAR-γ-Ligand anschließend maximal wirken kann. Eine geeignete Vorbehandlung mit einem RAR/RXR-Liganden, insbesondere mit Retinsäure, kann vorteilhafterweise über wenige Stunden bis einige Tage durchgeführt werden. Besonders bevorzugt sind 1 bis 3 Tage, insbesondere 2 Tage. Eine derartige Vorbehandlung kann beispielsweise durch eine entsprechende Infusion und/oder durch ein- oder mehrfache orale Verabreichung des entsprechenden Wirkstoffes erfolgen. Weiterhin kann es auch bevorzugt sein, zunächst den mindestens einen PPAR-γ-Liganden zu verabreichen, und den mindestens einen RAR/RXR-Liganden zeitversetzt zu applizieren. Eine gleichzeitige Verabreichung von Liganden für beide Rezeptorarten kann gleichfalls zum gewünschten Ergebnis führen und bevorzugt sein.

Als Liganden für den Rezeptor PPAR-γ werden bevorzugt Wirkstoffe aus der Klasse Thiazolidindione, insbesondere Ciglitazon, Pioglitazon, Rosiglitazon oder Mischungen davon, verwendet. Als Ligand für die Rezeptoren RAR/RXR wird bevorzugt Retinsäure und/oder mindestens ein pharmakologisch verträgliches Derivat davon, insbesondere *trans-*Retinsäure und/oder mindestens ein Derivat davon, eingesetzt. Dieses Derivat kann beispielsweise ein Salz oder ein Ester sein, insbesondere ein Ester mit einer Alkansäure mit vorzugsweise 1 bis 4 C-Atomen.

In einer weiteren Ausführungsform der Erfindung können neben den oben beschriebenen Liganden für PPAR-γ und RAR/RXR auch zusätzliche Stoffe, die auf die Aktivität der Wirkstoffe modulierend, insbesondere aktivierend wirken, eingesetzt werden. Vorzugsweise handelt es sich bei diesen Stoffen um Aktivatoren, insbesondere Co-Aktivatoren, die beispielsweise einen Komplex mit den beiden Rezeptortypen bilden können. Hierfür kommen Stoffe in Frage, die als weiterer Rezeptor an der Komplexbildung teilnehmen können und aktivierend auf die NIS-Genexpression im erfindungsgemäßen Sinne wirken.

Weiterhin kann durch Verabreichung geeigneter Stoffe die Wirkung von zelleigenen Stoffen, welche insbesondere als Suppressoren hemmend auf die Aktivierung der NIS-Genexpression durch PPAR-Liganden und RAR/RXR-Liganden wirken, abgeschwächt und/oder elimiert werden, so dass die NIS-Genexpression erfindungsgemäß gesteigert werden kann. Beispiele für solche, die Aktivierung der NIS-Genexpression hemmende Suppressoren sind der Leberlipidrezeptor (LXR) und/oder der Schilddrüsenhormonrezeptor (Ide T. et al., Molecular Endocrinolgy, Mai 2003; Behr M. and U. Loos, Exp Clin Endocrinol Diabetes 1996 (104) Suppl 4: 111-6). Diese Rezeptoren und/oder entsprechende Agonisten, z.B. Liganden dieser Rezeptoren, schwächen bzw. supprimieren die Heterodimerisierung von PPAR-γ und RAR/RXR. Durch Verabreichung von Antagonisten dieser supprimierend wirkenden Rezeptoren werden erfindungsgemäß diese Suppressoren ausgeschaltet bzw. blockiert und so die Interaktion von PPAR-γ und RAR/RXR verstärkt und damit deren erfindungsgemäße Wirkung auf die NIS-Genexpression bzw. -Funktion gesteigert. Als Antagonisten können beispielsweise solche Liganden für den Leberlipidrezeptor und/oder Schilddrüsenhormonrezeptor eingesetzt werden, die deren Aktivität blockieren. Beispiele für solche Antagonisten, insbesondere für Schilddrüsenhormonrezeptor-Antagonisten, sind TRIAC und/oder TETRAC.

Weiterhin kann als Aktivator beispielsweise auch ein Histondeacetylase-Inhibitor, insbesondere Trichostatin A und/oder ein Butyrat eingesetzt werden. Für Histondeacetylase-Inhibitoren ist bekannt, dass sie die NIS-Expression in einer mäßig differenzierten Schilddrüsenkarzinom-Zelllinie steigert (M. Kitazono et al., J Clin Endocrinol Metab. 2001 Jul. 86 (7): 3430-5). Alternativ können Suppressoren, welche insbesondere die Expression des NIS-Gens herunterregulieren, durch geeignete Substanzen ausgeschaltet bzw. blockiert werden.

Eine gezielte Induktion der NIS-Genexpression zur Diagnose und/oder Therapie der genannten Tumorarten kann erfindungsgemäß auch mit anderen Wirkstoffen erreicht werden. Diese Wirkstoffe können Hormone oder andere Liganden für bestimmte Rezeptoren sein, die auf der Oberfläche der Zellen der angegebenen Metastasen bzw. Karzinomen exprimiert werden. Als Beispiel für solche Wirkstoffe ist Prolaktin zu nennen, von dem bekannt ist, dass es in laktierenden Brustdrüsenzellen die Jodid-Aufnahme modulieren kann.

Eine besonders bevorzugte Ausführungsform des Diagnoseverfahrens umfasst eine *in vivo*-Diagnose. Hierfür wird mindestens ein Ligand für den Rezeptor PPAR-γ, z.B. ein Thiazolidindion wie beispielsweise Ciglitazon, und mindestens ein Ligand für den Rezeptor RAR- und/oder RXR, z.B. *trans*-Retinsäure, dem zu untersuchenden Patienten verabreicht. Die Verabreichung erfolgt bevorzugt oral oder parenteral. Eine intravenöse Applikation der Substanzen ist ebenfalls möglich. Vorzugsweise nach einer angemessenen Zeit, innerhalb derer die Induktion des NIS-Gens erfolgt, wird eine geeignete Dosis einer weiteren Substanz, insbesondere Technetium und/oder radioaktives Jod, z.B. ¹³¹Jodid, verabreicht. Diese Zeit beträgt vorteilhafterweise etwa 1 bis etwa 5 Tage, vorzugsweise etwa 1 bis etwa 3 Tage. Besonders bevorzugt sind etwa 2 Tage. Vorzugsweise nach einer weiteren angemessenen Zeitdauer, die zur Jodid-Aufnahme oder beispielsweise Technetium-Aufnahme in Zellen geeignet ist, wird die Aufnahme der Substanz in die Zellen analysiert. Dies geschieht beispielsweise durch ein Ganzkörperszintigramm oder ein lokales Szintigramm, beispielsweise der Brust, zum Nachweis von Primärtumoren und/oder Metastasen. Da die Jodid-Aufnahme nach einer solchen Behandlung erfindungsgemäß nur in NISexprimierenden Zellen potenziert wird, ermöglicht das *in vivo-*Diagnoseverfahren eine spezifische Indikation von Tumoren. Mit diesem Verfahren können selbst sehr kleine Tumore oder Mikrometastasen mit einem Durchmesser von weniger als 0,5 cm spezifisch erfasst werden, die beispielsweise noch kurativ behandelt werden können. Dies zeigt einen weiteren besonderen Vorteil des Verfahrens, denn der Nachweis derartiger Mikrometastasen ist mit herkömmlichen Diagnoseverfahren in der Regel nicht möglich.

Eine Analyse der oben genannten Krebsarten kann erfindungsgemäß nach einem in *vitro*-Diagnoseverfahren stattfinden. Dieses kann besonders bevorzugt für eine Ermittlung und/oder Optimierung der Dosis der zu applizierenden Wirkstoffe und/oder Substanzen, insbesondere der entsprechenden Rezeptorliganden, für die nachfolgende Therapie verwendet werden. Das *in vitro*-Diagnoseverfahren kann beispielsweise folgende Schritte umfassen. Die Zellen einer zu untersuchenden Probe, beispielsweise einer operativ resezierten Probe, werden zunächst mit beispielsweise mindestens einem Liganden für den Rezeptor PPAR-γ, z.B. einem Thiazolidindion, und mindestens einem Liganden für den Rezeptor RAR und/oder RXR, z.B. *trans*-Retinsäure, inkubiert. Anschließend werden so vorbereitete Zellen mit beispielsweise radioaktivem Jod und/oder Technetium behandelt. Die Bedingungen hierfür sollen eine Aufnahme des Jods oder Technetiums durch die Zellen erlauben und sind dem Fachmann bekannt. Schließlich kann der Gehalt bzw. die Konzentration des transportierten Jods bzw. Technetiums in den Zellen bestimmt werden. Die Messung des radioaktiven Jods bzw. Technetiums kann mittels bekannter Verfahren, wie beispielsweise in einem Gamma-Counter, durchgeführt werden. Alternativ kann statt Inkubieren mit einer Quelle für radioaktives Jod bzw. Technetium und anschließender Messung der Radioaktivität auch die Expression der NIS-mRNA in den Zellen der zu untersuchenden Probe nach der Behandlung mit den Wirkstoffen bzw. Liganden für die Rezeptoren PPAR-γ und RAR/RXR gemessen werden. Dabei kann die Bestimmung der Expression von NIS-mRNA mittels Reverser Transkriptase-Polymerasekettenreaktion (RT-PCR) oder anderer aus dem Stand der Technik bekannten Verfahren erfolgen.

Ein Therapieverfahren zur Behandlung von angegebenen Krebsarten kann beispielsweise folgende Schritte umfassen. Dem zu behandelnden Patienten werden zunächst Wirkstoffe verabreicht, die eine NIS-Genexpression in den genannten Tumorarten induzieren können. Besonders bevorzugt werden hierfür mindestens ein Ligand für den Rezeptor PPAR-γ, z.B. ein Thiazolidindion, in Kombination mit einem oder mehreren Liganden für den Rezeptor RAR und/oder RXR, z.B. *trans-*Retinsäure, verabreicht, insbesondere oral und/oder parenteral. Eine intravenöse Applikation der Wirkstoffe ist ebenfalls möglich. Die Verabreichung der Liganden kann zeitgleich oder zeitversetzt erfolgen. Nach einer angemessenen Zeit, innerhalb derer eine Induktion des NIS-Gens erfolgt, wird eine geeignete Dosis einer Substanz, die eine Radiolyse der Tumorzellen bewirken soll, verabreicht. Hierbei ist insbesondere radioaktives Jodid, z.B. ¹³¹Jodid, bevorzugt. Weder bei der Therapie noch bei den beschriebenen Diagnoseverfahren ist es zwingend erforderlich, dass bis zur Gabe von beispielsweise radioaktivem Jod eine angemessene Zeit abgewartet wird. Es ist ebenfalls möglich, dass die Verabreichung der Liganden und die Verabreichung von Jod beispielsweise zeitgleich erfolgen. Die beschriebenen Schritte werden nach Bedarf in geeigneten Zeitabständen z.B. zwischen 7 und 14 Tagen wiederholt. Die Radiojodid-Aufnahme erfolgt erfindungsgemäß organspezifisch vorwiegend in die Zellen der angegebenen Tumorarten, in denen durch die Behandlung eine Potenzierung der NIS-Expression und eine verstärkte Jodid-Aufnahme erfolgt. Im Gegensatz zu einer systemischen Radiotherapie, die aus dem Stand der Technik bekannt ist, werden mit dem Therapieverfahren die Tumorzellen benannter Karzinome und Metastasen gezielt angesprochen. Der Behandlungsfortgang und -erfolg kann beispielsweise mit den oben beschriebenen *in vivo-* und/oder *in vitro-*Diagnoseverfahren verfolgt werden. Mit besonderem Vorteil kann die Therapie nach einer operativen Entfernung von Primärtumoren erfolgen, um so erfolgreich Metastasen bekämpfen zu können. Unter Umständen kann es auch bevorzugt sein, die Therapie statt einer Operation oder eventuell vor einer Operation einzusetzen. Dies ist insbesondere dann vorteilhaft, wenn eine Operation aus irgendwelchen Gründen nicht in Frage kommt.

Bevorzugt enthalten Zusammensetzungen und Präparate als Wirkstoffe Liganden für die Rezeptoren PPAR, insbesondere PPAR-γ, und RAR und/oder RXR. Vorteilhaft sind dabei Zusammensetzungen, die mindestens einen Wirkstoff aus der Klasse Thiazolidindione und Retinsäure und/oder ihre Derivate, insbesondere die *trans*-Retinsäure, enthalten. Zusätzlich können solche Zusammensetzungen weitere Stoffe, vorzugsweise Aktivatoren, insbesondere Co-Aktivatoren, des NIS-Gens und/oder Histondeacetylase-Inhibitoren, welche ebenfalls stimulierend auf die NIS-Expression wirken, umfassen. Diesbezüglich wird auf die obige Beschreibung verwiesen. Die Zusammensetzungen oder-Präparate können ferner mindestens einen pharmakologisch unbedenklichen Träger- und/oder Hilfsstoff umfassen. Solche Zusammensetzungen bzw. Präparate können als Diagnostika und/oder als Medikamente zur Diagnose und/oder Behandlung von primären Karzinomen und/oder Metastasen, insbesondere von glandulären Karzinomen, vorzugsweise von Karzinomen der Schilddrüse, der Speicheldrüse, des Uterus und/oder von Mammakarzinomen sowie den entsprechenden Metastasen eingesetzt werden.

Die Erfindung umfasst weiterhin einen Kit, insbesondere für die Diagnose und/oder Therapie von NIS-Gen exprimierenden Karzinomen und/oder Metastasen, welcher Wirkstoffe enthält, die eine NIS-Genexpression in den Tumoren induzieren, stimulieren und/oder verstärken können. Dieser Kit enthält mindestens einen Liganden für den Rezeptor PPAR-γ, insbesondere ein Thiazolidindion wie beispielsweise Ciglitazon, und mindestens einen Liganden für den Rezeptor RAR-und/oder RXR, insbesondere eine *trans*-Retinsäure. Zusätzlich kann der Kit eine Quelle für radioaktives Jod und/oder Technetium enthalten. Weiterhin kann der Kit modulierend wirkende Stoffe, vorzugsweise Aktivatoren, insbesondere Co-Aktivatoren, enthalten. Diesbezüglich wird auf die obige Beschreibung verwiesen. Besonders bevorzugt ist hierbei ein Histondeacetylase-Inhibitor, insbesondere Trichostatin A oder ein Butyrat als Aktivator. Bezüglich weiterer Merkmale der erfindungsgemäßen Zusammensetzung und Präparate wird ebenfalls ausdrücklich auf die obige Beschreibung verwiesen.

Schließlich umfasst die Erfindung eine Verwendung von mindestens einem PPAR-Liganden, insbesondere einem PPAR-γ-Liganden, und mindestens einem RAR- und/oder RXR-Liganden zur Herstellung eines Diagnostikmittels zum Nachweis oder zur Herstellung eines Medikaments zur Behandlung von Karzinomen und/oder Metastasen, die ein NIS-Gen exprimieren. Bei diesen Karzinomen und/oder Metastasen handelt es sich insbesondere um Primärtumore und/oder Metastasen von glandulären Karzinomen, insbesondere von Speicheldrüsenkarzinomen, Schilddrüsenkarzinomen, Uteruskarzinomen und/oder Mammakarzinomen.

In einer bevorzugten Ausführungsform dieses Aspektes der Erfindung handelt es sich bei dem mindestens einen PPAR-γ-Liganden um ein oder mehrere Thiazolidindione, insbesondere um Ciglitazon, Pioglitazon und/oder Rosiglitazon. Bei dem mindestens einen RAR- und/oder RXR-Liganden handelt es sich vorteilhafterweise um Retinsäure, insbesondere *trans*-Retinsäure, und/oder ein oder mehrere pharmakologisch verträgliche Derivate davon. Ein entsprechendes pharmakologisch verträgliches Derivat ist vorteilhafterweise ein Salz und/oder ein Ester der Retinsäure, insbesondere ein Ester mit einer Alkansäure mit vorzugsweise 1 bis 4 C-Atomen.

Durch die Verwendung der beschriebenen Liganden wird die Expression des NIS-Gens induziert bzw. stimuliert und/oder verstärkt, so dass in den betroffenen Zellen letztendlich eine erhöhte Aktivität dieses Symporters und damit eine verstärkte Aufnahme von Jod oder von anderen Substanzen zu beobachten ist, für welche der Symporter eine Affinität aufweist. Diese verstärkte Aktivität des Symporters wird erfindungsgemäß für eine Diagnose oder eine Therapie ausgenutzt, indem die Aufnahme der affinen Substanz in die Zelle nachgewiesen wird, oder indem durch die Aufnahme von beispielsweise radioaktiven affinen Substanzen eine Radiolyse und damit eine Zerstörung der betroffenen Zellen bewirkt wird. Als affine Substanzen werden insbesondere Halogenide, und hierbei besonders bevorzugt Jod beansprucht. Als weitere affine Substanz ist beispielsweise Technetium bevorzugt.

In einer besonders bevorzugten Ausführungsform dieses Aspektes der erfindungsgemäßen Verwendung ist die radioaktive Substanz radioaktives Jod, vorzugsweise ¹²³I, ¹²⁵I und/oder ¹³¹I. Besonders bevorzugt ist es, wenn das Jod, insbesondere das radioaktive Jod, in Form eines Jodids, vorzugsweise in Form eines Alkali- und/oder Erdalkalijodids vorliegt. Besonders vorteilhaft ist hierbei Natriumjodid (Nal).

In einer weiteren vorteilhaften Ausführungsform der erfindungsgemäßen Verwendungen ist das Diagnostikmittel bzw. das Medikament derart ausgestaltet, dass der mindestens eine RAR/RXR-Ligand zuerst und der mindestens eine PPAR-Ligand nach einer angemessenen Zeit appliziert wird. Eine derartige zeitversetzte Applikation hat den Vorteil, dass die synergistische Wirkung beider Liganden bzw. beider Ligandengruppen verstärkt werden kann, was insbesondere daran liegen kann, dass der Rezeptor PPAR-γ zunächst beispielsweise durch die Gegenwart von Suppressoren in seiner Aktivität unterdrückt wird. Durch die Aktivierung von RAR/RXR durch den oder die entsprechenden Liganden wird auch PPAR-γ in eine aktive Form gebracht, so dass durch die nachfolgende Applikation von Liganden für PPAR-γ der volle synergistische Effekt erreicht werden kann. Andererseits kann es auch vorteilhaft sein, zunächst mindestens einen PPAR-Liganden, insbesondere mindestens einen PPAR-γ-Liganden, und später mindestens einen RAR/RXR-Liganden zu verabreichen. Auch die zeitgleiche Applikation der Ligandengruppen kann bevorzugt sein. Bezüglich weiterer Merkmale dieser erfindungsgemäßen Verwendungen wird ausdrücklich auf die obige Beschreibung verwiesen.

Neben der beschriebenen Verwendung der erwähnten Liganden zur Herstellung eines Diagnostikmittells bzw. zur Herstellung eines Medikaments umfasst die Erfindung ebenfalls die Verwendung von mindestens einem PPAR-Liganden, insbesondere einem PPAR-γ-Liganden, und mindestens einem RAR- und/oder RXR-Liganden zum diagnostischen Nachweis bzw. zur Behandlung von Karzinomen und/oder Metastasen, die ein NIS-Gen exprimieren. Bezüglich weiterer Merkmale dieses Aspektes der Erfindung wird auf die obige Beschreibung verwiesen.

Das beschriebene erfindungsgemäße Verfahren zur Diagnose der oben angegebenen Tumorarten sowie die Präparate und Verwendungen weisen gegenüber den bekannten Verfahren und Zusammensetzungen u.a. den Vorteil auf, dass sie eine spezifische Erkennung von Primärtumoren und/oder deren Metastasen sowie eine effektive und tumorspezifische Radiojodtherapie ermöglichen.

Weitere Vorteile, Merkmale und Anwendungsmöglichkeiten der Erfindung werden nachstehend anhand der Ausführungsbeispiele mit Bezug auf die Zeichnungen beschrieben. Hierbei können die verschiedenen Merkmale jeweils für sich oder in Kombination miteinander verwirklicht sein. In den Zeichnungen zeigen:
den bekannten Verfahren und Zusammensetzungen u.a. den Vorteil auf, dass sie eine spezifische Erkennung von Primärtumoren und/oder deren Metastasen sowie eine effektive und tumorspezifische Radiojodtherapie ermöglichen.

Weitere Vorteile, Merkmale und Anwendungsmöglichkeiten der Erfindung werden nachstehend anhand der Ausführungsbeispiele mit Bezug auf die Zeichnungen beschrieben. Hierbei können die verschiedenen Merkmale jeweils für sich oder in Kombination miteinander verwirklicht sein. In den Zeichnungen zeigen:
- **Fig. 1**: die Wirkung von CIG auf die tRA-induzierte Jodid-Aufnahme in Brustkrebszellen. Die Zellen wurden mit 1 µM tRA mit oder ohne 5 µM CIG über 48 h behandelt und der Jodid-Aufnahmetest mit (schwarze Rechtecke) oder ohne (weiße Rechtecke) 30 mM KClO₄ durchgeführt. Die Werte wurden als Mittelwerte ± SD (n=3) ausgedrückt;
- **Fig. 2**: die zellspezifische Wirkung von tRA und CIG auf die Jodid-Aufnahme. MCF-7-und HeLa-Zellen wurden mit 1 µM tRA mit oder ohne 5 µM CIG über 24 h behandelt. Der Jodid-Aufnahmetest wurde in Gegenwart oder Abwesenheit von 30 mM KClO₄ über 1 h durchgeführt. Die Werte wurden als Mittelwerte ± SD (n=3) ausgedrückt.

### Beispiele

Alle hier benutzten Zelllinien wurden von der Deutschen Sammlung von Mikroorganismen und Zellkulturen (DSMZ; Braunschweig, Deutschland, DSMZ-Nr.: MCF-7 (ACC 115)) bezogen. MCF-7 Zellen wurden in RPMI 1640-Medium mit 10% FCS (fetal calf serum) und Zusatz von 1 x nichtessentieller Aminosäuren, 0,01 mg/ml bovinem Insulin und 5% Penicillin/Streptomycin kultiviert. Die HeLa-Zellen wurden in RPMI 1640 Medium gezüchtet. Alle Reagenzien für die Zellkultur wurden, wenn nicht anders angegeben, von Invitrogen, Karlsruhe, Deutschland bezogen.

Die Experimente wurden mit dem gepaarten T-Test unter Benutzung des Programms von Statview, Version 5.0 (SAS Institute Inc. USA) statistisch ausgewertet.

### Jodid-Aufnahrne-Experimente

Zellen wurden in Platten mit 24 Vertiefungen (24 Well Plates) 24 h lang gezüchtet. Danach wurden die Zellen gewaschen und anschließend die zu testenden Substanzen (Ciglitazon, *trans*-Retinsäure mit oder ohne KClO₄) in frischem Medium zugegeben. Für die Jodid-Aufnahme wurden die Zellen zuerst zweimal mit vorgewärmten RPMI 1640 Medium ohne FCS gewaschen und dann in 1 ml RPMI 1640 ohne FCS, das 0,1 µCi/ml Na¹²⁵I (spezifische Aktivität: 10 mCi/mmol, Amersham Biosciences, Freiburg, Deutschland) sowie 10 µM Nal/Vertiefung enthielt, über 1 h lang inkubiert.

Nach der Inkubation wurden die Zellen zweimal mit eiskaltem RPMI 1640 gewaschen und in 2% SDS-PAGE-Probenpuffer (0,0625 M Tris-HCl, pH 6,8) mit 1 U/µl Benzonase (Merck KgaA, Darmstadt, Deutschland) lysiert. Die ¹²⁵I-Aufnahme wurde in einem Gamma-Counter gemessen. Ein Aliquot von jeder Probe wurde für die Bestimmung der Protein-Konzentration mit dem DC-Protein-Bestimmungskit von Bio-Rad (München, Deutschland) eingesetzt, um damit eine Normierung bei unterschiedlichen Zellzahlen zu erreichen.

MCF-7-Zellen wurden zuerst mit 1 µM *trans*-Retinsäure (tRA) (Sigma, München, Deutschland) in Gegenwart oder Abwesenheit von 5 µM CIG (Biomol, Hamburg, Deutschland) über 48 h zu unterschiedlichen Zeitintervallen behandelt. Die maximale Aufnahme wurde bei 1 µM tRA nach 48 h erreicht. Der Jodid-Aufnahmetest zeigte, dass die gleichzeitige Behandlung der Zellen mit tRA und CIG die durch tRA um das 5-fache gesteigerte Jodid-Aufnahme nochmals um das 3,3-fache ansteigen (potenzieren) lässt **(Fig. 1).**

Eine Induktion der Jodid-Aufnahme durch kombinierte Stimulation mit CIG und tRA wurde auch in HeLa-Zellen getestet, um die spezifische Wirkung dieser Substanzen auf MCF-7-Zellen aufzuzeigen. HeLa-Zellen wurden mit 1 µM tRA mit oder ohne 5 µM CIG über 48 h behandelt. Im Vergleich zu MCF-7-Zellen wurde keine Induktion der Jodid-Aufnahme in HeLa-Zellen nach 48 h Behandlung mit tRA, CIG sowie tRA kombiniert mit CIG beobacht (**Fig. 2**). Die gleichen Ergebnisse wurden erhalten, wenn HEK-293-Zellen in einem analogen Experiment eingesetzt wurden (Ergebnisse nicht gezeigt). Diese Ergebnisse zeigen die spezifische Wirkung von CIG in Kombination mit tRA in MCF-7-Zellen bezüglich der Jodid-Aufnahme.

Lebensfähigkeit der Zellen wurde durch die Behandlung durch CIG und tRA nicht beeinträchtigt, wie die Färbung mit Trypanblau zeigte (Ergebnisse nicht gezeigt).

### Blockierungsexperimente

Um die Spezifität der NIS-vermittelten Jodid-Aufnahme zu testen, wurden die im vorherigen Ausführungsbeispiel beschriebenen Experimente auch in Gegenwart von 30 mM KClO₄, dem stärksten spezifischen Inhibitor von NIS, durchgeführt.

Die Jodid-Aufnahme wurde durch KClO₄ inhibiert (**Fig. 1**), was auf eine spezifische NIS-vermittelten Jodid-Aufnahme durch die BrustkrebsZellen hindeutet.

Diese Ergebnisse zeigen, dass die tRA-induzierte Jodid-Aufnahme durch CIG um ein vielfaches verstärkt wird und dass dieses durch die spezifische Funktion von NIS vermittelt ist.

Die vorliegende Erfindung ist nicht auf die oben beschriebenen Anwendungsbeispiele beschränkt. Vielmehr sind viele Abwandlungen möglich, die durch Fachleute erzielbar sind, und damit vom Umfang dieser Erfindung erfasst sind.

### Literaturstellen

Carrasco N. Iodide transport in the thyroid gland. Biochim Biophys Acta. 1993 Jun 8;1154(1):65-82.
De La Vieja A, Dohan O, Levy O, Carrasco N. Molecular analysis of the sodium/iodide symporter: impact on thyroid and extrathyroid pathophysiology. Physiol Rev. 2000 Jul;80(3):1083-105.
Heufelder AE, Morgenthaler N, Schipper ML, Joba W. Sodium iodide symporter-based strategies for diagnosis and treatment of thyroidal and nonthyroidal malignancies. Thyroid. 2001 Sep;11 (9):839-47.
Inoue K, Kawahito Y, Tsubouchi Y, Yamada R, Kohno M, Hosokawa Y, Katoh D, Bishop-Bailey D, Hla T, Sano H. Expression of peroxisome proliferator-activated receptor (PPAR)-gamma in human lung cancer. Anticancer Res. 2001 Jul-Aug;21(4A):2471-6
Kilgore MW, Tate PL, Rai S, Sengoku E, Price TM. MCF-7 and T47D human breast cancer cells contain a functional peroxisomal response. Mol Cell Endocrinol. 1997 May 16;129(2):229-35.
Kitazono M, Robey R, Zhan Z, Sarlis NJ, Skarulis MC, Aikou T, Bates S, Fojo T. Low concentrations of the histone deacetylase inhibitor, depsipeptide (FR901228), increase expression of the Na(+)/I(-) symporter and iodine accumulation in poorlydifferentiated thyroid carcinoma cells. J Clin Endocrinol Metab. 2001 Jul;86(7):3430-5.
Kogai T, Schultz JJ, Johnson LS, Huang M, Brent GA. Retinoic acid induces sodium/iodide symporter gene expression and radioiodide uptake in the MCF-7 breast cancer cell line. Proc Natl Acad Sci USA. 2000 Jul 18;97(15):8519-24.
Mazzaferri EL, 1996 in The Thyroid, eds. Braverman LE and Utiger RD (Lippincott-Raven, Philadelphia), pp. 942-945.
Murphy GJ, Holder JC. PPAR-gamma agonists: therapeutic role in diabetes, inflammation and cancer. Trends Pharmacol Sci. 2000 Dec;21(12);469-74. Review.
Nwankwo JO, Robbins ME. Peroxisome proliferator-activated receptorgamma expression in human malignant and normal brain, breast and prostate-derived cells. Prostaglandins Leukot Essent Fatty Acids. 2001 Apr-May;64(4-5):241-5.
Riedel C, Dohan O, De la Vieja A, Ginter CS, Carrasco N. Journey of the iodide transporter NIS: from its molecular identification to its clinical role in cancer. Trends Biochem Sci. 2001 Aug;26 (8):490-6.
Sato M, Yajima Y, Kawashima S, Tanaka K, Kagechika H. Synergistic potentiation of thiazolidinedione-induced ST 13 preadipocyte differentiation by RAR synergists. Biochem Biophys Res Commun. 2001 Jan 26;280(3):646-51.
Sugiyama Y, Murase K, Ikeda H. Mechanisms of thiazolidinedione derivatives for hypoglycemic and insulin sensitizing effects. Nippon Rinsho. 2000 Feb;58(2):370-5. Review. Japanese.
Tazebay UH, Wapnir IL, Levy O, Dohan O, Zuckier LS, Zhao QH, Deng HF, Amenta PS, Fineberg S, Pestell RG, Carrasco N. The mammary gland iodide transporter is expressed during lactation and in breast cancer. Nat Med. 2000 Aug;6(8):871-8.
Titcomb MW, Gottardis MM, Pike JW, Allegretto EA. Sensitive and specific detection of retinoid receptor subtype proteins in cultured cell and tumor extracts. Mol Endocrinol. 1994 Jul;8(7):870-7.

## Patentansprüche

1. Verwendung von mindestens einem PPAR-γ-Liganden und mindestens einem RAR- und/oder RXR-Liganden zur Herstellung eines Diagnostikmittels zum Nachweis von Karzinomen und/oder Metastasen, die mindestens ein NIS-Gen exprimieren, **dadurch gekennzeichnet, dass** zum Nachweis der Karzinome und/oder Metastasen zusätzlich zu den genannten Liganden eine weitere Substanz, die durch den Symporter NIS aktiv transportiert wird, verabreicht wird.

2. Verwendung von mindestens einem PPAR-γ-Liganden und mindestens einem RAR- und/oder RXR-Liganden zur Herstellung eines Medikaments zur Behandlung von Karzinomen und/oder Metastasen, die mindestens ein NIS-Gen exprimieren, **dadurch gekennzeichnet, dass** zur Behandlung der Karzinome und/oder Metastasen zusätzlich zu den genannten Liganden eine weitere Substanz, die durch den Symporter NIS aktiv transportiert wird, verabreicht wird.

3. Verwendung nach Anspruch 1 oder Anspruch 2, **dadurch gekennzeichnet, dass** die Karzinome und/oder Metastasen Primärtumore und/oder Metastasen von glandulären Karzinomen, insbesondere Speicheldrüsenkarzinomen, Schilddrüsenkarzinomen, Uteruskarzinomen und/oder Mammakarzinomen, sind.

4. Verwendung nach einem der vorhergehenden Ansprüche, **dadurch gekennzeichnet, dass** der mindestens eine PPAR-γ-Ligand ein Thiazolidindion, insbesondere aus der Gruppe bestehend aus Ciglitazon, Pioglitazon, Rosiglitazon oder Mischungen davon, ist.

5. Verwendung nach einem der vorhergehenden Ansprüche, **dadurch gekennzeichnet, dass** der mindestens eine RAR- und/oder RXR-Ligand Retinsäure, insbesondere *trans*-Retinsäure, und/oder mindestens ein pharmakologisch verträgliches Derivat davon ist.

6. Verwendung nach Anspruch 5, **dadurch gekennzeichnet, dass** das pharmakologisch verträgliche Derivat ein Salz oder ein Ester, insbesondere ein Ester mit einer Alkansäure mit vorzugsweise 1 bis 4 C-Atomen, ist.

7. Verwendung nach einem der vorhergehenden Ansprüche, **dadurch gekennzeichnet, dass** das Diagnosemittel und/oder das Medikament ferner mindestens einen Stoff umfasst, der eine Stimulierung und/oder Verstärkung einer NIS-Genexpression durch die Wirkstoffe moduliert, insbesondere verstärkt, wobei vorzugsweise der Stoff mindestens einen Suppressor der NIS-Gen-expression, insbesondere einen Leberlipidrezeptor und/oder einen Schilddrüsenhormonrezeptor, antagoniert.

8. Verwendung nach einem der vorhergehenden Ansprüche, **dadurch gekennzeichnet, dass** die weitere Substanz ein Halogenid, vorzugsweise Jod, und/oder Technetium ist, wobei vorzugsweise das Jod in Form eines Alkali- und/oder Erdalkalijodids, vorzugsweise als Natriumjodid, vorliegt.

9. Verwendung nach einem der vorhergehenden Ansprüche, **dadurch gekennzeichnet, dass** die weitere Substanz eine radioaktive Substanz ist, insbesondere radioaktives Jod, vorzugsweise ¹²³I, ¹²⁵I und/oder ¹³¹I.

10. Verwendung nach einem der vorhergehenden Ansprüche, **dadurch gekennzeichnet, dass** Metastasen mit einem Durchmesser kleiner als etwa 1 cm, insbesondere kleiner als etwa 0,5 cm, diagnostizierbar und/oder behandelbar sind.

11. Verwendung nach einem der vorhergehenden Ansprüche, **dadurch gekennzeichnet, dass** eine Verabreichung des Diagnostikmittels und/oder des Medikaments so vorgesehen ist, dass der mindestens eine RAR- und/oder RXR-Ligand zuerst und der mindestens eine PPAR-Ligand nach einer angemessenen Zeit appliziert wird, insbesondere nach etwa einigen Stunden bis etwa einigen Tagen, vorzugsweise nach etwa 1 bis etwa 3 Tagen.

12. Verfahren zur *in vitro*-Diagnose von NIS-Gen exprimierenden Karzinomen und/oder Metastasen von Karzinomen, insbesondere von glandulären Karzinomen, vorzugsweise Speicheldrüsenkarzinomen, Schilddrüsenkarzinomen, Uteruskarzinomen und/oder Mammakarzinomen, welches mindestens die folgenden Schritte umfasst:
a) Inkubieren von Zellen einer zu untersuchenden Probe mit mindestens einem Liganden für den Rezeptor PPAR-γ und mindestens einem Liganden für den Rezeptor RAR und/oder RXR, die eine Induktion der NIS-Genexpression in Zellen von Karzinomen und/oder Metastasen zu stimulieren und/oder zu verstärken vermögen,
b) Inkubieren der im ersten Schritt erhaltenen Zellen mit mindestens einer Substanz, die durch den Symporter NIS in die Zellen aktiv transportiert werden kann, insbesondere mit radioaktivem Jod und/oder Technetium,
c) Bestimmen der Aufnahme der besagten Substanz durch die Zellen.

13. Verfahren zur *in vitro*-Diagnose von NIS-Gen exprimierenden Karzinomen und/oder Metastasen von Karzinomen, insbesondere von glandulären Karzinomen, vorzugsweise Speicheldrüsenkarzinomen, Schilddrüsenkarzinomen, Uteruskarzinomen und/oder Mammakarzinomen, welches mindestens die folgenden Schritte umfasst:
a) Inkubieren von Zellen einer zu untersuchenden Probe mit mindestens einem Liganden für den Rezeptor PPAR-γ und mindestens einem Liganden für den Rezeptor RAR und/oder RXR, die eine Induktion der NIS-Genexpression in Zellen von Karzinomen und/oder Metastasen zu stimulieren und/oder zu verstärken vermögen,
b) Bestimmen der Expression von NIS-mRNA durch die Zellen, insbesondere mittels Reverser Transkriptase-Polymerasekettenreaktion (RT-PCR).

14. Kombinationspräparat in Form eines Kits, umfassend räumlich voneinander getrennt eine Zusammensetzung, die mindestens ein PPAR-γ-Ligand und mindestens ein RAR- und/oder RXR-Ligand enthält, sowie mindestens eine weitere Substanz, die durch den Symporter NIS in die Zellen von Karzinomen und/oder Metastasen aktiv transportiert werden kann, zur getrennten, gegebenenfalls zeitlich abgestuften Anwendung zur Diagnose und/oder Therapie von NIS-Gen exprimierenden Karzinomen und/oder Metastasen, insbesondere von glandulären Karzinomen, vorzugsweise Speicheldrüsenkarzinomen, Schilddrüsenkarzinomen, Uterukarzinomen und/oder Mammakarzinomen.

15. Kombinationspräparat nach Anspruch 14, **dadurch gekennzeichnet, dass** der PPAR-γ-Ligand ein Thiazolidindion, insbesondere aus der Gruppe bestehend aus Ciglitazon, Pioglitazon, Rosiglitazon oder Mischungen davon, ist.

16. Kombinationspräparat nach Anspruch 14 oder Anspruch 15, **dadurch gekennzeichnet, dass** der RAR- und/oder RXR-Ligand Retinsäure, insbesondere *trans-*Retinsäure, und/oder mindestens ein pharmakologisch verträgliches Derivat davon ist.

17. Kombinationspräparat nach Anspruch 16, **dadurch gekennzeichnet, dass** das pharmakologisch verträgliche Derivat ein Salz oder ein Ester, insbesondere ein Ester mit einer Alkansäure mit vorzugsweise 1 bis 4 C-Atomen, ist.

18. Kombinationspräparat nach einem der Ansprüche 14 bis 17, **dadurch gekennzeichnet, dass** die Zusammensetzung ferner mindestens einen Stoff enthält, der die Stimulierung und/oder Verstärkung durch die Wirkstoffe moduliert, insbesondere verstärkt, wobei vorzugsweise der Stoff mindestens einen Suppressor der NIS-Genexpression, insbesondere einen Leberlipidrezeptor und/oder einen Schilddrüsenhormonrezeptor, antagoniert.

19. Kombinationspräparat nach einem der Ansprüche 14 bis 18, **dadurch gekennzeichnet, dass** die Zusammensetzung ferner einen Histondeacetylase-Inhibitor, insbesondere Trichostatin A und/oder ein Butyrat, enthält.

20. Kombinationspräparat nach einem der Ansprüche 14 bis 19, **dadurch gekennzeichnet, dass** die Zusammensetzung ferner mindestens einen pharmakologisch unbedenklichen Träger-und/oder Hilfsstoff umfasst.

21. Kombinationspräparat nach einem der Ansprüche 14 bis 20, **dadurch gekennzeichnet, dass** die Zusammensetzung zur oralen und/oder parenteralen Verabreichung vorgesehen ist.

22. Kombinationspräparat nach einem der Ansprüche 14 bis 21, **dadurch gekennzeichnet, dass** die weitere Substanz, die durch den Symporter NIS in die Zellen von Karzinomen und/oder Metastasen aktiv transportiert wird, ein Halogenid, insbesondere Jod, und/oder Technetium ist.

23. Kombinationspräparat nach einem der Ansprüche 14 bis 22, **dadurch gekennzeichnet, dass** die weitere Substanz, die durch den NIS-Symporter in die Zellen von Karzinomen und/oder Metastasen aktiv transportiert wird, radioaktiv ist.

24. Kombinationspräparat nach Anspruch 23, **dadurch gekennzeichnet, dass** die radioaktive Substanz das radioaktive Jod ¹²³I,¹²⁵I und/oder ¹³¹I ist, welches insbesondere in Form eines Alkali- oder Erdalkalijodids, vorzugsweise als Natriumjodid, vorliegt.

## Claims

1. Use of at least one PPAR-γ ligand and at least one RAR and/or RXR ligand for producing a diagnostic composition for detecting carcinomas and/or metastases which express at least one NIS gene, **characterized in that**, for detecting the carcinomas and/or metastases, a further substance which is actively transported by the NIS symporter is administered in addition to the ligands mentioned.

2. Use of at least one PPAR-γ ligand and at least one RAR and/or RXR ligand for producing a medicament for treating carcinomas and/or metastases which express at least one NIS gene, **characterized in that**, for treating the carcinomas and/or metastases, a further substance which is actively transported by the NIS symporter is administered in addition to the ligands mentioned.

3. Use according to Claim 1 or Claim 2, **characterized in that** the carcinomas and/or metastases are primary tumors and/or metastases of glandular carcinomas, in particular salivary gland carcinomas, thyroid carcinomas, uterine carcinomas and/or carcinomas of the breast.

4. Use according to any of the preceding claims, **characterized in that** the at least one PPAR-γ ligand is a thiazolidinedione, in particular from the group consisting of ciglitazone, pioglitazone, rosiglitazone or mixtures thereof.

5. Use according to any of the preceding claims, **characterized in that** the at least one RAR and/or RXR ligand is retinoic acid, in particular *trans*-retinoic acid, and/or at least one pharmacologically acceptable derivative thereof.

6. Use according to Claim 5, **characterized in that** the pharmacologically acceptable derivative is a salt or an ester, in particular an ester with an alkanoic acid preferably having 1 to 4 C atoms.

7. Use according to any of the preceding claims, **characterized in that** the diagnostic composition and/or the medicament additionally includes at least one substance which modulates, in particular enhances, a stimulation and/or enhancement of NIS gene expression by the active compounds, where the substance preferably antagonizes at least one suppressor of NIS gene expression, in particular a liver lipid receptor and/or a thyroid hormone receptor.

8. Use according to any of the preceding claims, **characterized in that** the further substance is a halide, preferably iodine, and/or technetium, where the iodine is preferably in the form of an alkali metal and/or alkaline earth metal iodide, preferably of sodium iodide.

9. Use according to any of the preceding claims, **characterized in that** the further substance is a radioactive substance, in particular radioactive iodine, preferably ¹²³I, ¹²⁵I and/or ¹³¹I.

10. Use according to any of the preceding claims, **characterized in that** metastases with a diameter of less than about 1 cm, in particular less than about 0.5 cm, can be diagnosed and/or treated.

11. Use according to any of the preceding claims, **characterized in that** the diagnostic composition and/or the medicament is intended to be administered so that the at least one RAR and/or RXR ligand is administered first and the at least one PPAR ligand is administered after an appropriate time, in particular after about some hours to about some days, preferably after about 1 to about 3 days.

12. Method for the *in vitro* diagnosis of NIS gene-expressing carcinomas and/or metastases of carcinomas, in particular of glandular carcinomas, preferably salivary gland carcinomas, thyroid carcinomas, uterine carcinomas and/or carcinomas of the breast, which includes at least the following steps:
a) incubation of cells of a sample to be investigated with at least one ligand for the PPAR-γ receptor and at least one ligand for the RAR and/or RXR receptor, which are able to stimulate and/or to enhance induction of NIS gene expression in cells of carcinomas and/or metastases,
b) incubation of the cells obtained in the first step with at least one substance which can be actively transported by the NIS symporter into the cells, in particular with radioactive iodine and/or technetium
c) determination of the uptake of said substance by the cells.

13. Method for the *in vitro* diagnosis of NIS gene-expressing carcinomas and/or metastases of carcinomas, in particular of glandular carcinomas, preferably salivary gland carcinomas, thyroid carcinomas, uterine carcinomas and/or carcinomas of the breast, which includes at least the following steps:
a) incubation of cells of a sample to be investigated with at least one ligand for the PPAR-γ receptor and at least one ligand for the RAR and/or RXR receptor, which are able to stimulate and/or to enhance induction of NIS gene expression in cells of carcinomas and/or metastases,
b) determination of the expression of NIS mRNA by the cells, in particular by means of reverse transcriptase polymerase chain reaction (RT-PCR).

14. Combination product in the form of a kit including spatially separated from one another a composition which comprises at least one PPAR-γ ligand and at least one RAR and/or RXR ligand, and at least one further substance which can be actively transported by the NIS symporter into the cells of carcinomas and/or metastases, for separate, where appropriate sequential, use for diagnosis and/or therapy of NIS gene-expressing carcinomas and/or metastases, in particular of glandular carcinomas, preferably salivary gland carcinomas, thyroid carcinomas, uterine carcinomas and/or carcinomas of the breast.

15. Combination product according to Claim 14, **characterized in that** the PPAR-γ ligand is a thiazolidinedione, in particular from the group consisting of ciglitazone, pioglitazone, rosiglitazone or mixtures thereof.

16. Combination product according to Claim 14 or Claim 15, **characterized in that** the RAR and/or RXR ligand is retinoic acid, in particular *trans*-retinoic acid, and/or at least one pharmacologically acceptable derivative thereof.

17. Combination product according to Claim 16, **characterized in that** the pharmacologically acceptable derivative is a salt or an ester, in particular an ester with an alkanoic acid preferably having 1 to 4 C atoms.

18. Combination product according to any of Claims 14 to 17, **characterized in that** the composition additionally comprises at least one substance which modulates, in particular enhances, the stimulation and/or enhancement by the active compounds, where the substance preferably antagonizes at least one suppressor of NIS gene expression, in particular a liver lipid receptor and/or a thyroid hormone receptor.

19. Combination product according to any of Claims 14 to 18, **characterized in that** the composition additionally comprises a histone deacetylase inhibitor, in particular trichostatin A and/or a butyrate.

20. Combination product according to any of Claims 14 to 19, **characterized in that** the composition additionally includes at least one pharmacologically acceptable carrier and/or excipient.

21. Combination product according to any of Claims 14 to 20, **characterized in that** the composition is intended for oral and/or parenteral administration.

22. Combination product according to one of Claims 14 to 21, **characterized in that** the further substance which is actively transported by the NIS symporter into the cells of carcinomas and/or metastases is a halide, in particular iodine, and/or technetium.

23. Combination product according to any of Claims 14 to 22, **characterized in that** the further substance which is actively transported by the NIS symporter into the cells of carcinomas and/or metastases is radioactive.

24. Combination product according to Claim 23, **characterized in that** the radioactive substance is radioactive iodine ¹²³I, ¹²⁵I and/or ¹³¹I, which is in particular in the form of an alkali metal or alkaline earth metal iodide, preferably of sodium iodide.

## Revendications

1. Utilisation d'au moins un ligand de PPAR-γ et d'au moins un ligand de RAR et/ou de RXR pour préparer un agent de diagnostic destiné à la détection de carcinomes et/ou de métastases qui expriment au moins un gène NIS, **caractérisée en ce que**, pour la détection des carcinomes et/ou des métastases, outre les ligands mentionnés, on administre une substance supplémentaire, qui est transportée d'une manière active par le symporteur NIS.

2. Utilisation d'au moins un ligand de PPAR-γ et d'au moins un ligand de RAR et/ou de RXR pour préparer un médicament destiné au traitement de carcinomes et/ou de métastases qui expriment au moins un gène NIS, **caractérisée en ce que**, pour le traitement des carcinomes et/ou des métastases, outre les ligands mentionnés, on administre une substance supplémentaire, qui est transportée d'une manière active par le symporteur NIS.

3. Utilisation selon la revendication 1 ou 2, **caractérisée en ce que** les carcinomes et/ou les métastases sont des tumeurs primaires et/ou des métastases de cancers glandulaires, en particulier de cancers des glandes salivaires, de cancers de la glande thyroïde, de cancers de l'utérus et/ou de cancers mammaires.

4. Utilisation selon l'une des revendications précédentes, **caractérisée en ce que** l'au moins un ligand de PPAR-γ est une thiazolidinedione, en particulier du groupe consistant en la ciglitazone, la pioglitazone, la rosiglitazone ou les mélanges de celles-ci.

5. Utilisation selon l'une des revendications précédentes, **caractérisée en ce que** l'au moins un ligand de RAR et/ou de RXR est l'acide rétinoïque, en particulier l'acide trans-rétinoïque, et/ou en est au moins un dérivé acceptable d'un point de vue pharmacologique.

6. Utilisation selon la revendication 5, **caractérisée en ce que** le dérivé acceptable d'un point de vue pharmacologique est un sel ou un ester, en particulier un ester d'un acide alcanoïque ayant de préférence 1 à 4 atomes de carbone.

7. Utilisation selon l'une des revendications précédentes, **caractérisée en ce que** l'agent de diagnostic et/ou le médicament comprend en outre au moins une substance qui module une stimulation et/ou un renforcement d'une expression du gène NIS par les principes actifs, et en particulier les renforce, la substance antagonisant de préférence au moins un suppresseur de l'expression du gène NIS, en particulier un récepteur des lipides hépatiques et/ou un récepteur des hormones thyroïdiennes.

8. Utilisation selon l'une des revendications précédentes, **caractérisée en ce que** la substance supplémentaire est un halogénure, de préférence l'iode, et/ou le technétium, l'iode se présentant de préférence sous forme d'un iodure d'un métal alcalin et/ou alcalino-terreux, de préférence sous forme de l'iodure de sodium.

9. Utilisation selon l'une des revendications précédentes, **caractérisée en ce que** la substance supplémentaire est une substance radioactive, en particulier l'iode radioactif, de préférence le ¹²³I, le ¹²⁵I et/ou le ¹³¹I.

10. Utilisation selon l'une des revendications précédentes, **caractérisée en ce qu'**elle permet de diagnostiquer et/ou de traiter des métastases ayant un diamètre inférieur à environ 1 cm, et en particulier inférieur à environ 0,5 cm.

11. Utilisation selon l'une des revendications précédentes, **caractérisée en ce qu'**une administration de l'agent de diagnostic et/ou du médicament est prévue de telle sorte que l'au moins un ligand de RAR et/ou de RXR soit administré en premier, et l'au moins un ligand de PPAR soit administré au bout d'un temps approprié, en particulier au bout d'environ quelques heures à environ quelques jours, de préférence au bout d'environ 1 à environ 3 jours.

12. Procédé de diagnostic in vitro de carcinomes et/ou de métastases de carcinomes exprimant le gène NIS, en particulier de cancers glandulaires, de préférence de cancers des glandes salivaires, de cancers de la glande thyroïde, de cancers de l'utérus et/ou de cancers mammaires, qui comprend au moins les étapes suivantes :
a) incubation de cellules d'un échantillon devant être étudié à l'aide d'au moins un ligand pour le récepteur PPAR-γ et d'au moins un ligand pour le récepteur RAR et/ou RXR, qui permettent de stimuler et/ou de renforcer une induction de l'expression du gène NIS dans des cellules de carcinomes et/ou de métastases,
b) incubation des cellules obtenues dans la première étape avec au moins une substance qui peut être transportée d'une manière active dans les cellules grâce au symporteur NIS, en particulier avec de l'iode radioactif et/ou du technétium,
c) détermination de l'absorption, par les cellules, de ladite substance.

13. Procédé pour le diagnostic in vitro de carcinomes et/ou de métastases de carcinomes exprimant le gène NIS, en particulier de cancers glandulaires, de préférence de cancers des glandes salivaires, de cancers de la glande thyroïde, de cancers de l'utérus et/ou de cancers mammaires, qui comprend au moins les étapes suivantes :
a) incubation de cellules d'un échantillon devant être étudié, avec au moins un ligand pour le récepteur PPAR-γ et au moins un ligand pour le récepteur RAR et/ou RXR, qui permettent une stimulation et/ou un renforcement d'une induction de l'expression du gène NIS dans des cellules de carcinomes et/ou de métastases,
b) détermination de l'expression de l'ARNm-NIS par les cellules, en particulier par une réaction en chaîne par polymérase avec transcriptase inverse (RT-PCR).

14. Préparation en combinaison sous forme d'une trousse comprenant, spatialement écartées l'une de l'autre, une composition qui contient au moins un ligand de PPAR-γ et au moins un ligand de RAR et/ou de RXR, ainsi qu'au moins une substance supplémentaire, qui peut être transportée d'une manière active par le symporteur NIS dans les cellules de carcinomes et/ou de métastases, destinée à une utilisation séparée, éventuellement étagée dans le temps, et servant au diagnostic et/ou au traitement de carcinomes et/ou de métastases exprimant le gène NIS, en particulier de cancers glandulaires, de préférence de cancers des glandes salivaires, de cancers de la glande thyroïde, de cancers de l'utérus et/ou de cancers mammaires.

15. Préparation en combinaison selon la revendication 14, **caractérisée en ce que** le ligand de PPAR-γ est une thiazolidinedione, en particulier du groupe consistant en la ciglitazone, la pioglitazone, la rosiglitazone ou les mélanges de celles-ci.

16. Préparation en combinaison selon la revendication 14 ou 15, **caractérisée en ce que** le ligand de RAR et/ou de RXR est l'acide rétinoïque, en particulier l'acide trans-rétinoïque, et/ou en est au moins un dérivé acceptable d'un point de vue pharmacologique.

17. Préparation en combinaison selon la revendication 16, **caractérisée en ce que** le dérivé acceptable d'un point de vue pharmacologique est un sel ou un ester, en particulier un ester d'un acide alcanoïque ayant de préférence 1 à 4 atomes de carbone.

18. Préparation en combinaison selon l'une des revendications 14 à 17, **caractérisée en ce que** la composition comprend en outre au moins une substance qui module la stimulation et/ou le renforcement par les principes actifs, et en particulier les renforce, de préférence la substance antagonisant au moins un suppresseur de l'expression du gène NIS, en particulier un récepteur des lipides hépatiques et/ou un récepteur des hormones thyroïdiennes.

19. Préparation en combinaison selon l'une des revendications 14 à 18, **caractérisée en ce que** la composition contient en outre un inhibiteur d'histone-désacétylase, en particulier la trichostatine A et/ou un butyrate.

20. Préparation en combinaison selon l'une des revendications 14 à 19, **caractérisée en ce que** la composition comprend en outre au moins un support et/ou adjuvant inoffensif d'un point de vue pharmacologique.

21. Préparation en combinaison selon l'une des revendications 14 à 20, **caractérisée en ce que** la composition est prévue pour une administration orale et/ou parentérale.

22. Préparation en combinaison selon l'une des revendications 14 à 21, **caractérisée en ce que** la substance supplémentaire qui est transportée d'une manière active par le symporteur NIS dans les cellules de carcinomes et/ou de métastases est un halogénure, en particulier l'iode, et/ou le technétium.

23. Préparation en combinaison selon l'une des revendications 14 à 22, **caractérisée en ce que** la substance supplémentaire qui est transportée d'une manière active par le symporteur NIS dans les cellules de carcinomes et/ou de métastases est radioactive.

24. Préparation en combinaison selon la revendication 23, **caractérisée en ce que** la substance radioactive est l'iode radioactif ¹²³I, ¹²⁵I et/ou ¹³¹I, qui se présente en particulier sous forme d'un iodure d'un métal alcalin ou alcalino-terreux, de préférence sous forme de l'iodure de sodium.
